# EUROPEAN PATENT APPLICATION

(11) **EP 3 632 558 A1**
(43) Date of publication of application: **08.04.2020**
(21) Application number: 18810519.1
(22) Date of filing: 31.05.2018
(51) Int. Cl.: B01J 35/02, A61L 9/00, B01J 29/08, B01J 29/40, B01J 29/65, B01J 29/70, B01J 37/10

(54) **PHOTOCATALYST STRUCTURE, PHOTOCATALYST STRUCTURE COMPOSITION, PHOTOCATALYST COATING MATERIAL, PRODUCTION METHOD OF PHOTOCATALYST STRUCTURE, AND DECOMPOSITION METHOD OF ALDEHYDES**

(30) Priority: 31.05.2017 JP 2017108636; 31.05.2017 JP 2017108637; 31.05.2017 JP 2017108638
(71) Applicant: Furukawa Electric Co., Ltd., Chiyoda-ku Tokyo 100-8322 (JP)
(72) Inventor: MASUDA, Takao, Hokkaido 0600808 (JP); NAKASAKA, Yuta, Hokkaido 0600808 (JP); YOSHIKAWA, Takuya, Hokkaido 0600808 (JP); KATO, Sadahiro, Tokyo 100-8322 (JP); FUKUSHIMA, Masayuki, Tokyo 100-8322 (JP); TAKAHASHI, Hiroko, Tokyo 100-8322 (JP); BANBA, Yuichiro, Tokyo 100-8322 (JP); SEKINE, Kaori, Tokyo 100-8322 (JP)
(74) Representative: Gunzelmann, Rainer
(86) International application number: PCT/JP2018/021090
(87) International publication number: WO 2018/221702

(57) **Abstract**

An object of the present invention is to provide a structured photocatalyst that can effectively prevent aggregation of photocatalyst particles and maintain favorable photocatalytic functionality over a long period of time. A structured photocatalyst including a support of porous structure including a zeolite-type compound and at least one photocatalytic substance present in the support, the support including channels connecting with each other, and the photocatalytic substance including metal oxide nanoparticles and being present at least at the channels of the support.

## Description

### Technical Field

The present invention relates to a structured photocatalyst, a structured photocatalyst composition, a photocatalyst coated material, a method for producing a structured photocatalyst, and a method for decomposing aldehydes.

### Background Art

Photocatalysts, which exhibit actions of decompose organic materials and of imparting superhydrophilic effect to surfaces under photoirradiation, are used for environmental purification, anti-contamination, anti-bacterial, and anti-fogging applications, for example. As such photocatalysts, a photocatalyst including, for example, a metal oxide such as titanium oxide is widely used in a photocatalyst market due to reasons such as high photocatalytic activity, low cost, and high chemical stability.

In general, catalysts having greater area of active surfaces are more catalytically active, thus catalyst particles having smaller particle size are more desirable. This is also the case for the photocatalysts. Unfortunately, in case of nanoparticles of a metal oxide such as titanium oxide or the like, for example, particles are likely to aggregate to each other in a random manner. Such an aggregation (sintering) contributes to lowering of photocatalytic functionality.

To address the above issue of aggregation, in Patent Document 1, a method is disclosed in which a crystalline superstructure (titanium oxide mesocrystal) of meso size (specifically, approximately from 1 to 10 µm), in which titanium oxide nanocrystals are arranged in a regular manner, is used as a photocatalyst.

Such a titanium oxide mesocrystal can prevent nanoparticles or nanocrystals from aggregating in a random manner. However, the titanium oxide mesocrystals themselves are crystals of a large size (1 µm or greater), in which the nanocrystals are arranged in a regular manner, thus the specific surface area is smaller than that of typical titanium oxide nanoparticles (titanium oxide nanoparticle P25, a representative photocatalyst, has a specific surface area of approximately 50 m²/g), and has less catalytic activity compared to the catalytic activity of highly dispersed nanocrystal particles. In addition, titanium oxide mesocrystals are crystals of relatively large size, and do not easily aggregate compared to the nanoparticles. However, in case of titanium oxide mesocrystal as well, the particles are also brought into contact with each other, and may aggregate when used for long period of time.

### Citation List

### Patent Literature

Patent Document 1: WO 2013/115213 A1

### Summary of Invention

### Technical Problem

An object of the present invention is to provide a structured photocatalyst, a structured photocatalyst composition, a photocatalyst coated material, a method for producing a structured photocatalyst, and a method for decomposing aldehydes that enable to effectively prevent aggregation of photocatalyst particles and to maintain favorable photocatalytic functionality over a long period of time.

### Solution to Problem

As a result of intensive research to achieve the object described above, the present inventors have discovered that a structured photocatalyst can be obtained, which includes a support of porous structure including a zeolite-type compound and at least one photocatalytic substance present in the support, the support including channels connecting with each other, and the photocatalytic substance including metal oxide nanoparticles and being present at least at the channel of the support, thus effectively preventing aggregation of photocatalyst particles to maintain favorable photocatalytic functionality over a long period of time. Thus, the present inventors have completed the present invention based on such a discovery.

That is, the summary configurations of the present invention are as follows.
[1] A structured photocatalyst including a support of porous structure including a zeolite-type compound, and at least one photocatalytic substance present in the support, the support including channels connecting with each other, and the photocatalytic substance including a metal oxide nanoparticle and being present at least at the channels of the support.
[2] The structured photocatalyst according to [1], wherein the channels include an enlarged pore portion, and the photocatalytic substance is present at least at the enlarged pore portion.
[3] The structured photocatalyst according to [2], wherein the enlarged pore portion causes a plurality of pores constituting any one of the one-dimensional pore, the two-dimensional pore, and the three-dimensional pore to connect with each other.
[4] The structured photocatalyst according to any one of [1] to [3], wherein the metal oxide nanoparticles include titanium or an alloy oxide including titanium.
[5] The structured photocatalyst according to any one of [1] to [4], wherein the metal oxide nanoparticles have an average particle diameter that is greater than an average inner diameter of the channels and not greater than an inner diameter of the enlarged pore portion.
[6] The structured photocatalyst, for hydrodesulfurization, according to any one of [1] to [5], wherein the metal oxide nanoparticles include a metal element (M) in an amount from 0.5 mass% to 2.5 mass% with respect to the structured catalyst.
[7] The structured photocatalyst according to any one of [1] to [6], wherein
   the metal oxide nanoparticles have an average particle size from 0.1 nm to 50 nm.
[8] The structured photocatalyst, for hydrodesulfurization, according to [7], wherein
   the metal oxide nanoparticles have an average particle size ranging from 0.45 nm to 14.0 nm.
[9] The structured photocatalyst according to any one of [1] to [8], wherein
   a ratio of an average particle size of the metal oxide nanoparticles with respect to an average inner diameter of the channels is from 0.06 to 500.
[10] The structured photocatalyst according to [9], wherein
   the ratio of the average particle size of the metal oxide nanoparticles with respect to the average inner diameter of the channels is from 0.1 to 45.
[11] The structured photocatalyst according to [10], wherein
   the ratio of the average particle size of the metal oxide nanoparticles with respect to the average inner diameter of the channels is from 1.7 to 4.5.
[12] The structured photocatalyst according to any one of [1] to [11], wherein
   the channels include any one of a one-dimensional pore, a two-dimensional pore, and a three-dimensional pore defined by a framework of the zeolite-type compound, and an enlarged pore portion being different from any of the one-dimensional pore, the two-dimensional pore, and the three-dimensional pore,
   the channels have an average inner diameter from 0.1 mm to 1.5 nm, and the enlarged pore portion has an inner diameter from 0.5 mm to 50 nm.
[13] The structured photocatalyst according to any one of [1] to [12], further including
   at least one another photocatalytic substance held on an outer surface of the support.
[14] The structured photocatalyst according to [13], wherein
   a content of the at least one photocatalytic substance present in the support is greater than a content of the at least one another photocatalytic substance held on the outer surface of the support.
[15] The structured photocatalyst according to any one of [1] to [14], wherein
   the zeolite-type compound includes a silicate compound.
[16] The structured photocatalyst according to any one of [1] to [15], wherein
   the structured photocatalyst is dispersed in a dispersion medium.
[17] A structured photocatalyst composition comprising
   the structured photocatalyst described in any one of [1] to [16] and a binder material.
[18] The structured photocatalyst composition according to [17], wherein
   the binder material includes an organic binder.
[19] A photocatalyst coated material including:
   a base material; and
   a photocatalytic layer formed on the base material, wherein
   the photocatalytic layer includes the structured photocatalyst described in any one of [1] to [16].
[20] The photocatalyst coated material according to [19], wherein
   the base material is a building material.
[21] A method for producing a structured photocatalyst, including:
   sintering a precursor material (B), wherein a metal containing solution is impregnated into a precursor material (A) for forming a support of porous structure composed of a zeolite-type compound to form the precursor material (B); and
   hydrothermal-treating a precursor material (C) obtained by the sintering of the precursor material (B).
[22] The method for producing a structured photocatalyst according to [21], wherein
   From 50 to 500 mass% of a non-ionic surfactant in proportion with the precursor material (A) is added to the precursor material (A) prior to the sintering
[23] The method for producing a structured photocatalyst according to [21] or [22], wherein
   the metal containing solution is added to the precursor material (A) in portions in a plurality of times prior to the sintering to impregnate the precursor material (A) with the metal containing solution.
[24] The method for producing a structured photocatalyst according to any one of [21] to [23], wherein
   when impregnating the precursor material (A) with the metal containing solution prior to the sintering,
   an amount of the metal containing solution added to the precursor material (A) is adjusted to make a ratio (atomic ratio, Si/M) of silicon (Si) constituting the precursor material (A) relative to a metal element (M) included in the metal containing solution added to the precursor material (A) to be from 10 to 1000.
[25] The method for producing a structured photocatalyst according to [21], wherein
   in the hydrothermal-treating, the precursor material (C) is mixed with a structure directing agent.
[26] The method for producing a structured photocatalyst according to [21], wherein
   the hydrothermal-treating is performed under a basic condition.
[27] A method for decomposing aldehydes, wherein the aldehydes are decomposed using the photocatalyst described in any one of [1] to [16].

### Advantageous Effects of Invention

The present invention provides a structured photocatalyst including a support of porous structure including a zeolite-type compound, and at least one photocatalytic substance present in the support, the support including channels connecting with each other, and the photocatalytic substance including a metal oxide nanoparticle and being present at least at the channels of the support, thus making it possible to effectively prevent aggregation of photocatalyst particles and to maintain favorable photocatalytic functionality over a long period of time. The present invention also provides a structured photocatalyst composition, a photocatalyst coated material, a method for producing a structured photocatalyst, and a method for decomposing aldehydes.

### Brief Description of Drawings

FIG. 1 is a schematic view illustrating a structured photocatalyst according to an embodiment of the present invention, comprehensively illustrating an inner structure of the structured photocatalyst, where FIG. 1A is a perspective view (partially illustrated in a cross-section), and FIG. 1B is a partially enlarged cross-sectional view.
FIG. 2 is a partial enlarged cross-sectional view illustrating an example of catalytic functionality of a structured photocatalyst in FIG. 1.
FIG. 3 is a flowchart illustrating an example of a method for producing a structured photocatalyst in FIG. 1.
FIG. 4 is a schematic view illustrating a modified example of a structured photocatalyst in FIG. 1.
FIG. 5 is a cross-sectional view schematically illustrating an example of a photocatalyst coated material according to an embodiment of the present invention.

### Description of Embodiments

Hereinafter, embodiments of the present invention will be described in detail with reference to drawings.

### Configuration of Structured photocatalyst

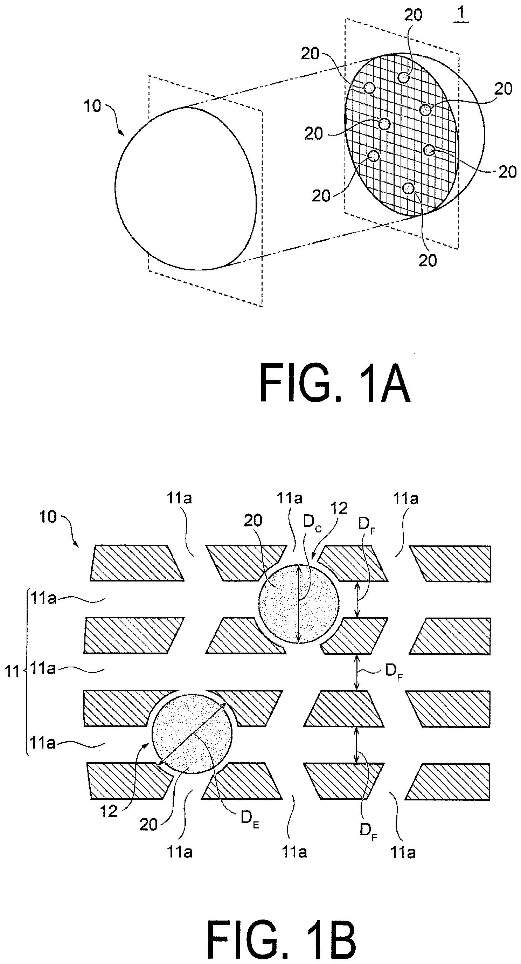

FIG. 1 is a schematic view of a configuration of a structured photocatalyst according to an embodiment of the present invention. FIG. 1A is a perspective view (partially illustrated in cross-section), and FIG. 1B is a partially enlarged cross-sectional view. Note that the structured photocatalyst in FIG. 1 indicates an example of the structured photocatalyst, and the shape, dimension, and the like of each of the configurations according to the present invention are not limited to those illustrated in FIG. 1.

As illustrated in FIG. 1A, a structured photocatalyst 1 includes a support 10 of porous structure including a zeolite-type compound, and at least one photocatalytic substance 20 present in the support 10.

In the structured photocatalyst 1, a plurality of photocatalytic substances 20, 20, ... are included within the porous structure of the support 10. The photocatalytic substance 20 is a substance having photocatalytic activity, and is specifically a metal oxide nanoparticle. The metal oxide nanoparticle will be described below in detail.

The support 10 is a porous structure, and as illustrated in FIG. 1B, a plurality of pores 11a, 11a, ... are preferably formed, and thus the support 10 includes channels 11 connecting with each other. Here, the photocatalytic substance 20 is present at least at the channel 11 of the support 10, and is preferably held at least at the channel 11 of a skeletal body 10.

Such a configuration restricts movement of the photocatalytic substance 20 within the support 10, to effectively prevent aggregation between the photocatalytic substances 20 and 20. This effectively prevents a decrease in the effective surface area as the photocatalytic substance 20, allowing the photocatalytic substance 20 to sustain the photocatalytic activity over a long period of time. That is, the structured photocatalyst 1 prevents deterioration in photocatalytic activity due to aggregation of the photocatalytic substance 20, to thus extend the life time of the structured photocatalyst 1. In addition, the extended life time of the structured photocatalyst 1 enables to reduce replacement frequency of the structured photocatalyst 1, making it possible to significantly reduce an amount of waste of the structured photocatalyst 1 that has been used, and to thus achieve resource savings.

In general, in case of using the structured photocatalyst in a fluid (e.g., air, water, or the like), the structured photocatalyst may be subjected to an external force from the fluid. In this case, when the photocatalytic substance is merely held in a state of only being deposited to an outer surface of the support 10, the photocatalytic substance may be easily disengaged from the outer surface of the support 10 due to an influence of the external force from the fluid. In contrast, in the structured photocatalyst 1, the photocatalytic substance 20 is present at least at the channels 11 of the support 10, thus, even if the photocatalytic substance 20 is subjected to an external force from the fluid, the photocatalytic substance 20 is less likely to be disengaged from the support 10. That is, in a case where the structured photocatalyst 1 is within the fluid, the fluid flows through the pores 11a of the support 10 into the channels 11, thus the flow rate of the fluid flowing through the channels 11 may be less than the flow rate of the fluid flowing on the outer surface of the support 10 due to a flow path resistance (frictional force). Due to an influence of such a flow path resistance, the pressure from the fluid onto the photocatalytic substance 20 being present within the channels 11 becomes lower than a pressure that the photocatalytic substance receives from the fluid at an exterior portion (external surface) of the support 10. This effectively prevents the disengagement of the photocatalytic substance 20 present in the support 11, allowing the photocatalytic substance 20 to stably sustain the photocatalytic activity over a long period of time. Note that such a flow path resistance may become greater, when the channels 11 of the support 10 includes a plurality of bends and branches, and the interior of the support 10 forms more complex, three-dimensional steric structure.

In addition, it is preferred that the channels 11 includes any one of a one-dimensional pore, a two-dimensional pore, and a three-dimensional pore defined by a framework of the zeolite-type compound, and an enlarged pore portion 12 being different from any of the one-dimensional pore, the two-dimensional pore, and the three-dimensional pore. In this case, the photocatalytic substance 20 is preferably present at least at the enlarged pore portion 12, and is more preferably enclosed at least within the enlarged pore portion 12. Here, the term one-dimensional pore refers to a pore of tunnel-type or cage-type that forms a one-dimensional channel, or a plurality of pores of tunnel-type or cage-type (a plurality of one-dimensional channels) that form a plurality of one-dimensional channels. In addition, the term two-dimensional pore refers to a two-dimensional channel in which a plurality of one-dimensional channels are two-dimensionally connected, and the term three-dimensional pore refers to a three-dimensional channel in which a plurality of one-dimensional channels are three-dimensionally connected.

This further restricts movement of the photocatalytic substance 20 within the support 10, making it possible to further effectively prevent disengagement of the photocatalytic substance 20 and aggregation between the photocatalytic substances 20 and 20. Here, the term inclusion refers to a state where the photocatalytic substance 20 is enclosed within the support 10. The photocatalytic substance 20 and the support 10 need not necessarily be in direct contact with each other, where the photocatalytic substance 20 may be indirectly held by the support 10 with other substances (e.g., a surfactant or the like) being interposed between the photocatalytic substance 20 and the support 10.

FIG. 1B illustrates a case where the photocatalytic substance 20 is included within the enlarged pore portion 12, however, the photocatalytic substance 20 is not solely limited to this configuration, where the photocatalytic substance 20 may be held by the channels 11 in a state of partially protruding outward of the enlarged pore portion 12. The photocatalytic substance 20 may also be partially embedded within a portion of the channels 11 other than the enlarged pore portion 12 (e.g., an inner wall portion of the channels 11), or may be held by fixing or the like.

In addition, the enlarged pore portion 12 may cause the plurality of pores 11a and 11a constituting any one of the one-dimensional pore, the two-dimensional pore, and the three-dimensional pore to connect with each other. This allows a separate channel different from the one-dimensional pore, the two-dimensional pore, or the three-dimensional pore to be provided inside of the support 10, to thus enable functionalities of the catalytic substance 20 to be further exhibited.

In addition, the channels 11 is three-dimensionally formed including a branch portion or a merging portion within the support 10, and the enlarged pore portion 12 is preferably provided at the branch portions or the merging portions of the channel 11.

An average inner diameter D_{F} of the channels 11 formed in the support 10 is calculated from the average lengths of the minor axes and the major axes of the pores 11a constituting any one of the one-dimensional pore, the two-dimensional pore, and the three-dimensional pore, and is, for example, from 0.1 nm to 1.5 nm, and preferably from 0.5 nm to 0.8 nm. In addition, an inner diameter D_{E} of the enlarged pore portion 12 is, for example, from 0.5 nm to 50 nm, preferably from 1.1 nm to 40 nm, and more preferably from 1.1 nm to 3.3 nm. The inner diameter D_{E} of the enlarged pore portion 12 varies, for example, depending on a fine pore diameter of the precursor material (A) described below and an average particle size D_{C} of the photocatalytic substances 20 that are included. The inner diameter D_{E} of the enlarged pore portion 12 has a size which enables inclusion of the photocatalytic substance 20.

The support 10 includes a zeolite-type compound. Examples of the zeolite-type compound include, for example, silicate compounds such as zeolites (aluminosilicate salts), cation exchanged zeolites, and silicalite; zeolite analog compounds such as aluminoborate salts, aluminoarsenate salts, and germanate salts; and phosphate-based zeolite analog materials such as molybdenum phosphates. In particular, the zeolite-type compound preferably includes a silicate compound.

The framework of the zeolite-type compound is selected from FAU type (Y type or X type), MTW type, MFI type (ZSM-5), FER type (ferrierite), LTA type (A type), MWW type (MCM-22), MOR type (mordenite), LTL type (L type), BEA type (beta type), and the like. Preferably, the framework is MFI type, and is more preferably ZSM-5. In the zeolite-type compound, a plurality of pores are formed, each of which has a pore diameter corresponding to the respective framework. For example, the maximum pore diameter of the MFI type is 0.636 nm (6.36 Å) and the average pore diameter is 0.560 nm (5.60 Å).

The photocatalytic substance 20 will be described below in detail.

The photocatalytic substance 20 includes a metal oxide nanoparticle. There are cases where the metal oxide nanoparticles 20 are primary particles, and cases where the metal oxide nanoparticles 20 are secondary particles including aggregated primary particles. The average particle size D_{C} of the metal oxide nanoparticles 20 is preferably greater than the average inner diameter D_{F} of the channels 11 and not greater than the inner diameter D_{E} of the enlarged pore portion 12 (D_{F} < D_{C} ≤ D_{E}). Such metal oxide nanoparticles 20 are preferably included within the enlarged pore portion 12 inside the channels 11, to restrict movement of the metal oxide nanoparticles 20 within the support 10. Thus, even if the metal oxide nanoparticles 20 are subjected to the external force from a fluid, the movement of the metal oxide nanoparticles 20 within the support 10 is suppressed to make it possible to effectively prevent the metal oxide nanoparticles 20, 20, ... included within the respective enlarged pore portions 12, 12, ... dispersed at the channels 11 of the support 10 from coming into contact with each other.

Further, the average particle size D_{C} of the metal oxide nanoparticles 20, for both the primary particle and the second particle, is preferably from 0.1 nm to 50 nm, more preferably not less than 0.1 nm and less than 30 nm, and still more preferably from 0.45 nm to 14.0 nm, and particularly preferably from 1.0 nm to 3.3 nm. Further, a ratio (D_{C}/D_{F}) of the average particle size Dc of the metal oxide nanoparticles 20 with respect to the average inner diameter D_{F} of the channels 11 is preferably from 0.06 to 500, more preferably from 0.1 to 45, still more preferably from 1.1 to 45, and particularly preferably from 1.7 to 4.5.

In addition, in a case where the photocatalytic substance 20 includes a metal oxide nanoparticles, a metal element (M) of the metal oxide nanoparticles is preferably included in from 0.5 to 2.5 mass% with respect to the structured photocatalyst 1, and more preferably in from 0.5 to 1.5 mass% with respect to the structured photocatalyst 1. For example, when the metal element (M) is Co, the content of the Co element (mass%) is represented by {(mass of Co element)/(mass of all elements of the structured catalyst 1)}×100.

The metal oxide nanoparticle described above is required to be constituted by metal oxide, where, for example, the metal oxide nanoparticles may be constituted by single metal oxide, or may be constituted by a mixture of two or more types of metal oxides. Note that in the present specification, the "metal oxide" constituting the metal oxide nanoparticles (as raw material) refers to an oxide containing one type of metal element (M) and a composite oxide containing two or more types of metal elements (M), and is a generic term for an oxide containing one or more types of metal elements (M).

Examples of such a metal oxide include, for example, cobalt oxide (CoOₓ), nickel oxide (NiOₓ), iron oxide (FeOₓ), copper oxide (CuOₓ), zirconium oxide (ZrOₓ), cerium oxide (CeOₓ), aluminum oxide (AlOₓ), niobium oxide (NbOₓ), titanium oxide (TiOₓ), bismuth oxide (BiOₓ), molybdenum oxide (MoOₓ), vanadium oxide (VOₓ), chromium oxide (CrOₓ), tungsten oxide (WOₓ), and cerium oxide (CeOₓ), where any one or more types of oxides is preferably included as a major component. In particular, titanium oxide and alloy oxides containing these are more preferred, and titanium oxide is more preferred. Furthermore, in a case where the metal oxide nanoparticles contain metal oxides constituted by two or more types of the metal species described above, the metal oxides can be referred to as composite metal oxide.

In addition, a ratio (atomic ratio Si/M) of silicon (Si) constituting the support 10 with respect to a metal element (M) constituting the metal oxide nanoparticles 20 is preferably from 10 to 1000, and more preferably from 50 to 200. When the ratio described above is greater than 1000, an action as a photocatalytic substance may not be sufficiently exhibited, for example, the activity is low. On the other hand, when the ratio is less than 10, the proportion of the metal oxide nanoparticles 20 becomes too large, and the strength of the support 10 tends to decrease. Note that the metal oxide nanoparticles 20 mentioned above, which is a metal oxide nanoparticles held within the support 10 or supported by the support 10, does not include a metal oxide nanoparticle adhering to the outer surface of the support 10.

### Photocatalytic Activity of Structured Photocatalyst

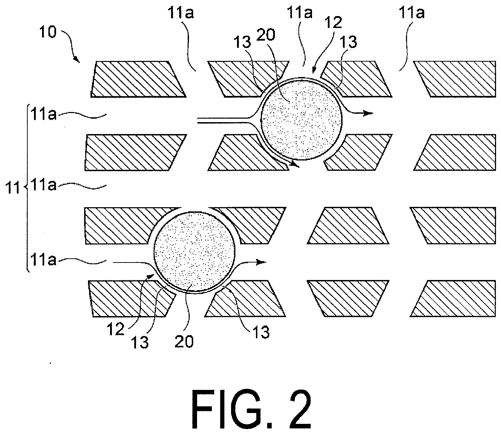

FIG. 2 is a partial enlarged cross-sectional view illustrating an example of catalytic functionality of a structured photocatalyst 1. As illustrated in FIG. 2, the metal oxide nanoparticles 20, which are a photocatalytic substance, are held by the channels 11 (in particular, the enlarged pore portions 12) of the support 10. Thus, for example, moisture (H₂O) and oxygen (O₂) in the atmosphere flow through the pore 11a into the channels 11 to come into contact with the metal oxide nanoparticles 20 held within the channel 11. Further, light (in particular, ultraviolet light) irradiated on the outer surface of the structured photocatalyst 1 partially passes through the support 10 or enters from the channels 11 to reach the metal oxide nanoparticles 20 held within the channels 11, while being reflected within the channels 11. Then, when the moisture (H₂O) and oxygen (O₂) flowing into the channels 11 come into contact with the metal oxide nanoparticles 20, oxidizing agents such as OH⁻ and O²⁻ are generated due to photocatalytic functionality of the metal oxide nanoparticles 20 activated by light (in particular, ultraviolet light) irradiated for a certain period of time. These oxidizing agents can decompose bacteria, odor components, contamination components, and the like, to thus exhibit anti-contamination properties (e.g., degradation power).

In addition, in the structured photocatalyst 1, movement of the metal oxide nanoparticles 20 within the support 10 is restricted such that aggregation between the metal oxide nanoparticles 20 and 20 is effectively prevented. This effectively prevents a decrease in the effective surface area as a photocatalyst, allowing the metal oxide nanoparticles 20 to sustain the photocatalytic functionality over a long period of time. That is, with the structured photocatalyst 1, a decrease in the photocatalytic functionality due to aggregation of the photocatalyst particles can be effectively prevented to thus extend the life time as the structured photocatalyst 1. In addition, the structured photocatalyst 1 has an extended life time, thereby reducing replacement frequency of the structured photocatalyst 1. Thus, an amount of waste of the structured photocatalyst 1 that has been used can be reduced significantly, and to contribute resource savings.

In a case where the average particle size D_{C} of the metal oxide nanoparticles 20 is greater than the average inner diameter D_{F} of the channels 11 and less than the inner diameter D_{E} of the enlarged pore portion 12 (D_{F} < D_{C} < D_{E}), a small channel 13 (the arrow in the figure) is formed between the metal oxide nanoparticle 20 and the enlarged pore portion 12, and the moisture (H₂O) and oxygen (O₂) flowing into the small channel 13 are brought into contact with the metal oxide nanoparticle 20. Then, the metal oxide nanoparticle 20, while being restricted in the movement by being enclosed by the enlarged pore portion 12, can maintain a large contact area with a fluid (e.g., the atmosphere) including moisture (H₂O) and oxygen (O₂) flowing into the channel 11.

### Modified Example of Structured Photocatalyst 1

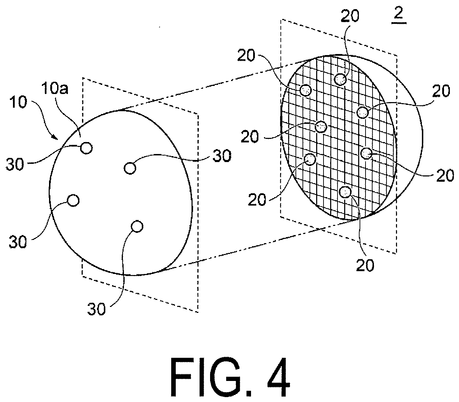

FIG. 4 is a schematic view illustrating a modified example of the structured photocatalyst 1 in FIG. 1.

The structured photocatalyst 1 in FIG. 1 illustrates a case of the structured photocatalyst 1 including the support 10 and the photocatalytic substance 20 present in the support 10, however, the structured photocatalyst 1 is not solely limited to this configuration, and, for example, as illustrated in FIG. 4, the structured photocatalyst 2 may further include at least one another photocatalytic substance 30 held on an outer surface 10a of the support 10.

The photocatalytic substance 30 is a substance that exhibits photocatalytic activity. The materials of the another photocatalytic substance 30 may be identical to or different from the materials of the photocatalytic substance 20. According to this configuration, a content of a photocatalytic substance held by the structured photocatalyst 2 can be increased to further promote a photocatalytic activity of the photocatalytic substance.

In this case, the content of the photocatalytic substance 20 present in the support 10 is preferably greater than a content of the at least one another photocatalytic substance 30 held on the outer surface 10a of the support 10.

This allows the photocatalytic activity of the photocatalytic substance 20 held within the support 10 to be dominant, to thus exhibit the photocatalytic activity in a stable manner.

### Method for Producing Structured Photocatalyst

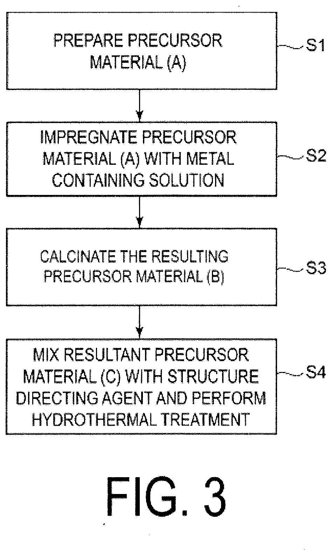

FIG. 3 is a flowchart illustrating an example of a method for producing the structured photocatalyst 1 in FIG. 1. An example of the method for producing the structured photocatalyst will be described below.

### Step S1: Preparation Step

As illustrated in FIG. 3, a precursor material (A) is firstly prepared for obtaining a support of the porous, structure including the zeolite-type compound. The precursor material (A) is preferably a regular mesoporous material, and can be appropriately selected in accordance with the type (composition) of the zeolite-type compound constituting the support of the structured photocatalyst.

Here, when the zeolite-type compound constituting the support of the structured photocatalyst is a silicate compound, the regular mesoporous material is preferably a compound including a Si-O skeleton in which pores having fine pore diameter of from 1 to 50 nm are uniformly sized and regularly developed one-dimensionally, two-dimensionally, or three-dimensionally. Such a regular mesoporous material is obtained as a variety of synthetic materials depending on the synthetic conditions, where specific examples of the synthetic material include, for example, SBA-1, SBA-15, SBA-16, KIT-6, FSM-16, and MCM-41, where in particular, MCM-41 is preferred. Note that the fine pore diameter of SBA-1 is from 10 to 30 nm, the fine pore diameter of SBA-15 is from 6 to 10 nm, the fine pore diameter of SBA-16 is 6 nm, the fine pore diameter of KIT-6 is 9 nm, the fine pore diameter of FSM-16 is from 3 to 5 nm, and the fine pore diameter of MCM-41 is from 1 to 10 nm. In addition, examples of such a regular mesoporous material include, for example, mesoporous silica, mesoporous aluminosilicate, and mesoporous metallosilicate.

The precursor material (A) may be a commercially available product or a synthetic product. A publicly known method for synthesizing a regular mesoporous material can be conducted in case of synthesizing the precursor material (A). For example, a mixed solution containing a raw material that contains constituent elements of the precursor material (A) and a molding agent for defining the structure of the precursor material (A) is prepared, and the pH is adjusted as necessary and a hydrothermal treatment (hydrothermal synthesis) is performed. Subsequently, a precipitate (product) obtained by the hydrothermal treatment is recovered (e.g., filtered), washed and dried as necessary, and is further subjected to a calcination treatment to obtain the precursor material (A) that is a regular mesoporous material in a powdery form. Here, examples of a solvent for the mixed solution that can be used include, for example, water, an organic solvent such as alcohol, or a mixed solvent of these. Further, the raw material, which is selected in accordance with the type of the support, and examples of the raw material include, for example, silica agents such as tetraethoxysilane (TEOS); fumed silica; and quartz sand. In addition, various types of surfactants, block copolymers, and the like can be used as the molding agent, which are preferably selected depending on the type of the synthetic material of the regular mesoporous material. A surfactant such as hexadecyltrimethylammonium bromide is preferred when producing MCM-41, for example. The hydrothermal treatment can be performed under treatment conditions at from 0 to 2000 kPa at from 80 to 800°C for from 5 hours to 240 hours within a sealed container. For example, a calcination treatment can be performed under treatment conditions in air, at from 350 to 850°C for from 2 hours to 30 hours.

### Step S2: Impregnation Step

The precursor material (A) prepared as above is then impregnated with a metal containing solution to obtain the precursor material (B).

The metal containing solution is a solution containing a metal component (e.g., a metal ion) corresponding to the metal element (M) constituting the metal oxide nanoparticle of the structured photocatalyst, and can be prepared, for example, by dissolving a metal salt containing the metal element (M) in a solvent. Examples of such a metal salt include, for example, chlorides, hydroxides, oxides, sulfates, and nitrates, and in particular, nitrates are preferred. Examples of the solvent that can be used include, for example, water, an organic solvent such as alcohol, or a mixed solvent of these.

The method for impregnating the precursor material (A) with the metal containing solution is not particularly limited; however, for example, the metal containing solution is preferably added in portions in a plurality of times while stirring the precursor material (A) in a powdery form prior to the calcination step described below. In addition, a surfactant is preferably added to the precursor material (A) as an additive before adding the metal containing solution to the precursor material (A) from the viewpoint of facilitating infiltration of the metal containing solution into the pores of the precursor material (A). It is believed that such additives serve to cover the outer surface of the precursor material (A) and inhibit the subsequently added metal-containing solution from adhering to the outer surface of the precursor material (A), making it easier for the metal-containing solution to enter the pores of the precursor material (A).

Examples of such an additive include, for example, non-ionic surfactants such as polyoxyethylene alkyl ether including polyoxyethylene oleyl ether, and polyoxyethylene alkylphenyl ether. These surfactants, which cannot infiltrate into the pores of the precursor material (A) due to their large molecular size, do not adhere to the interior of the pores. Thus, these surfactants may not prevent the infiltration of the metal containing solution into the pores. As the method for adding the non-ionic surfactant, for example, it is preferred to add from 50 to 500 mass% of the non-ionic surfactant to the precursor material (A) prior to the calcination step described below. When an added amount of the non-ionic surfactant to the precursor material (A) is less than 50 mass%, the preventing action hardly occur, and when more than 500 mass% of the non-ionic surfactant is added to the precursor material (A), the viscosity becomes excessively high, which is not preferred. Thus, the added amount of the non-ionic surfactant to the precursor material (A) is set to a value within the range described above.

In addition, an added amount of the metal containing solution added to the precursor material (A) is preferably adjusted as appropriate in view of the amount of a metal element (M) contained in the metal containing solution with which the precursor material (A) is impregnated (i.e., the amount of the metal element (M) to be present in the precursor material (B)). For example, prior to the calcination step described below, the added amount of the metal containing solution added to the precursor material (A) is adjusted to make a ratio (atomic ratio Si/M) of silicon (Si) constituting the precursor material (A) relative to a metal element (M) included in the metal containing solution added to the precursor material (A) to be from 10 to 1000, and more preferably from 50 to 200. For example, in a case where a surfactant is added to the precursor material (A) as an additive before adding the metal containing solution to the precursor material (A), the added amount of the metal containing solution added to the precursor material (A) is adjusted to make the atomic ratio Si/M to be from 50 to 200, thus the metal element (M) of the metal oxide nanoparticle is included in from 0.5 to 2.5 mass% of the structured photocatalyst. In the state of the precursor material (B), an amount of a metal element (M) present within the pores is generally proportional to the added amount of the metal containing solution added to the precursor material (A), provided that the metal concentration of the metal containing solution, the presence or absence of the additive, and other conditions such as temperature, pressure, and the like are the same. The amount of the metal element (M) present in the precursor material (B) also has a proportional relationship with the amount of metal element constituting the metal oxide nanoparticle present in the support of the structured photocatalyst. Accordingly, the added amount of the metal containing solution added to the precursor material (A) is controlled to the range described above, then the metal containing solution can be sufficiently impregnated into the fine pores of the precursor material (A), and, by extension, the amount of metal oxide nanoparticle to be present in the support of the structured photocatalyst can be adjusted.

After impregnating the precursor material (A) with the metal containing solution, a washing treatment may be performed as necessary. Examples of a washing solution that can be used include, for example, water, an organic solvent such as alcohol, or a mixed solvent of these. Furthermore, after the precursor material (A) is impregnated with the metal containing solution and the washing treatment is performed as necessary, it is preferred that a drying treatment is further performed. Examples of the drying treatment include overnight air drying and high temperature drying at 150°C or lower. Note that when the calcination treatment described below is performed in a state where the moisture included in the metal containing solution and water in the washing solution is still present in the precursor material (A) in a large amount, the skeletal structure of the precursor material (A) serving as the regular mesoporous material may break down, and thus it is preferred to perform the drying treatment thoroughly.

### Step S3: Calcination step

Next, the precursor material (B), which is the precursor material (A) for forming the support of porous structure including a zeolite-type compound is impregnated with the metal containing solution, is subjected to a calcination treatment to obtain the precursor material (C).

For example, the calcination treatment is preferably performed under treatment conditions in air, at from 350 to 850°C for 2 hours to 30 hours. With such a calcination treatment, the metal component impregnated into the regular mesoporous material undergoes crystal growth to form a metal oxide nanoparticle within the pore.

### Step S4: Hydrothermal Treatment Step

Next, a mixed solution of the precursor material (C) and the structure directing agent is prepared, and the precursor material (C) obtained by sintering the precursor material (B) is subjected to hydrothermal treatment to obtain a structured photocatalyst.

The structure directing agent is a molding agent for defining a skeletal structure of the support of the structured photocatalyst, where, for example, a surfactant can be used. The structure directing agent is preferably selected according to the skeletal structure of the support of the structured photocatalyst, where, for example, a surfactant such as tetramethylammonium bromide (TMABr), tetraethylammonium bromide (TEABr), or tetrapropylammonium bromide (TPABr) is preferably used.

The mixing of the precursor material (C) with the structure directing agent may be performed during this hydrothermal treatment step or may be performed prior to the hydrothermal treatment step. Furthermore, the method for preparing the mixed solution is not particularly limited, where the precursor material (C), the structure directing agent, and a solvent may be mixed simultaneously, or the precursor material (C) and the structure directing agent are each dispersed in a solvent in the solution independently, then the dispersion solutions may be mixed with each other. Examples of the solvent that can be used include, for example, water, an organic solvent such as alcohol, or a mixed solvent of these. In addition, the pH of the mixed solution is preferably adjusted using an acid or a base before performing the hydrothermal treatment.

The hydrothermal treatment can be performed by a publicly known method, and, for example, the hydrothermal treatment can be preferably performed under a treatment condition at from 0 to 2000 kPa at from 80 to 800°C for from 5 hours to 240 hours within a sealed container. In addition, the hydrothermal treatment is preferably performed under a basic condition. Although the reaction mechanism here is not necessarily apparent, a hydrothermal treatment is performed using the precursor material (C) as a raw material to cause the skeletal structure of the precursor material (C) as a regular mesoporous material becomes increasingly disintegrated. However, due to an action of the structure directing agent, a new skeletal structure (porous structure) is formed as a support of the structured photocatalyst, while the positions of the metal oxide nanoparticles are maintained within the pores of the precursor material (C). The structured photocatalyst obtained in this way includes a support of porous structure and a metal oxide nanoparticle present in the support, and the support further has a channel in which a plurality of pores connect with each other due to the porous structure, and at least a part of the metal oxide nanoparticles is held at the channel of the support.

In addition, in the hydrothermal treatment step in this embodiment, a mixed solution in which the precursor material (C) and the structure directing agent are mixed is prepared, and then the precursor material (C) is subjected to the hydrothermal treatment. However, the hydrothermal treatment step is not limited thereto, and the precursor material (C) may be subjected to the hydrothermal treatment without the precursor material (C) being mixed with the structure directing agent.

The precipitate (structured photocatalyst) obtained after the hydrothermal treatment is preferably washed, dried, and sintered as necessary after recovery (e.g., filtration). Examples of the washing solution that can be used include, for example, water, an organic solvent such as alcohol, or a mixed solvent of these. Examples of the drying treatment include overnight air drying and high temperature drying at 150°C or lower. Note that when a calcination treatment is performed in the state where there is a large amount of moisture remaining in the precipitate, the skeletal structure as a support of the structured photocatalyst may disintegrate, and thus it is preferred to perform the drying treatment thoroughly. For example, the calcination treatment can also be performed under treatment conditions in air, at from 350 to 850°C for from 2 hours to 30 hours. Such a calcination treatment burns out the structure directing agent adhering to the structured photocatalyst. The structured photocatalyst can also be used as-is without subjecting the recovered precipitate to a calcination treatment, depending on the purpose of use. For example, when the environment under which the structured photocatalyst is used is a high temperature environment of an oxidizing atmosphere, exposing the structured photocatalyst to a usage environment for a certain period of time allows the structure directing agent to be burned out and to obtain a structured photocatalyst similar to that when subjected to the calcination treatment. Thus, the resultant structured photocatalyst can be used as-is. In addition, for example, in the method for producing a structured photocatalyst composition and a method for producing a photocatalyst coated material described below, the structured photocatalyst is mixed with a binder material to form a coating material, and then the coating material is applied to a base body. In a case where a heat treatment is thereafter performed on the base body to form the photocatalytic layer, the structure directing agent is burned out to form a structured photocatalyst, similar to the case where the calcination treatment is performed. Thus, the resultant structured photocatalyst can be used as-is.

### Structured Photocatalyst Composition

Incidentally, when actually using the photocatalyst, a photocatalytic layer is generally formed on a surface of a base material (e.g., a wall material, a tile, a glass (mirror), a circulation filtration device, or a sanitary pottery). Examples of the method for forming such a photocatalytic layer include a method in which an organic binder is mixed with a photocatalyst to form a coating material, and the resultant coating material is applied onto a surface of a base material (JP 2007-519799 T and JP 2012-210557 A, and a method in which a photocatalyst is fixed onto a binding agent layer formed beforehand on a base material (JP 2006-021994 A).

However, in such methods for forming a photocatalytic layer in the related art, a configuration is employed in which photocatalyst particles are in direct contact with the binder material used for fixing, the organic binder may be decomposed due to high catalytic activity of the photocatalyst. Such a decomposition of the binder material leads to a deterioration of the coating material (photocatalyst composition), or contributes to a separation of the photocatalyst of the photocatalytic layer formed on a surface of the base material, from the surface of the base material.

As a result of intensive research to achieve the object described above, the present inventors have discovered that a structured photocatalyst composition can be obtained, which includes a support of porous structure containing a structured photocatalyst and a binder material, the structured photocatalyst being constituted by a zeolite-type compound, and at least one photocatalytic substance present in the support, the support including channels connecting with each other, and the photocatalytic substance including a metal oxide nanoparticle and being present at least at the channel of the support, thus preventing a direct contact between the binder material and the photocatalytic substance to prevent decomposition of the binder material due to the photocatalytic substance, to maintain favorable photocatalytic functionality over a long period of time. The structured photocatalyst composition according to this embodiment will be described below in detail.

The structured photocatalyst composition according to the present embodiment mainly contains the structured photocatalyst according to the embodiment described above, and a binder material. Note that the structured photocatalyst composition may further contains various additives such as solvents as necessary.

According to such a configuration, the photocatalytic substance is included within the support of porous structure which is constituted by a zeolite-type compound, and mixed with the binder material described below as a structured photocatalyst. Thus, in the structured photocatalyst composition, the binder material is effectively prevented from coming in direct contact with the photocatalytic substance, thus enabling to prevent decomposition of the binder material due to the photocatalytic substance. As a result, the binder material does not deteriorate over a long period of time, the stability as a coating material is improved, and even after a photocatalytic layer is formed on a surface of a base material, the photocatalytic layer can sustain superior catalytic activity without being disengaged from the surface of the base material.

Furthermore, in such a structured photocatalyst 1, movement of the photocatalytic substance 20 within the support 10 is restricted, to effectively prevent aggregation between the photocatalytic substances 20, 20. This effectively prevents a decrease in the effective surface area as the photocatalytic substance 20, allowing the photocatalytic substance 20 to sustain the photocatalytic activity over a long period of time. That is, the structured photocatalyst 1 prevents a decline in the photocatalytic activity due to aggregation of the photocatalytic substances 20, to extend the life time as the structured photocatalyst 1. In addition, the extended life time of the structured photocatalyst 1 enables to reduce replacement frequency of the structured photocatalyst 1, making it possible to significantly reduce an amount of waste of the structured photocatalyst 1 that has been used, and to achieve resource savings.

Note that in the structured photocatalyst composition, the another photocatalytic substance 30 described above and illustrated in FIG. 4, which is present at the outer surface 10a of the support 10, thus comes into direct contact with the binder material. This may cause the photocatalytic substance 30 to decompose the binder material. Thus, the content of the at least one another photocatalytic substance 30 held on the outer surface 10a of the support 10 is preferably less than the content of the photocatalytic substance 20 present in the support 10.

### Method for Producing Structured Photocatalyst Composition

The structured photocatalyst composition can be produced by mixing the structured photocatalyst and the binder material, and by adding various additives as necessary.

The method of mixing is not particularly limited, and mixing can be performed by a publicly known method. For example, mixing can be performed by stirring and mixing, or by kneading using a three-roll mill or a high pressure homogenizer. Note that the structured photocatalyst includes a support including porous structure, thus, from the viewpoint of maintaining the structure in a favorable manner, the structured photocatalyst is preferably mixed by a method which does not involve a high pressure physically, such as stirring and mixing,

In addition, the form of the structured photocatalyst composition is not particularly limited, and may be a coating material or a film form. In a case where the photocatalyst composition is, for example, a coating material, the viscosity of the coating material is preferably adjusted as appropriate in accordance with its usage mode (spray coating, coating by brushing, dipping, printing, or the like).

### 1. Structured Photocatalyst

As a structured photocatalyst, the structured photocatalyst produced by the production method described above is used.

### 2. Binder material

A binder material that is used may be an organic binder or an inorganic binder.

In the photocatalyst composition in the related art, an inorganic binder is preferably used as a binder material to avoid decomposition of the binder material by the photocatalytic substance. However, in the structured photocatalyst composition according to the present embodiment, the photocatalytic substance is included within the support serving as a structured photocatalyst, thus the photocatalytic substance is effectively prevented from coming in direct contact with the binder material. Thus, decomposition of the binder material due to the photocatalytic substance can be prevented. Therefore, in the structured photocatalyst composition according to this embodiment, an organic binder can be preferably used as well.

Examples of the organic binder that can be used include polyurethanes, poly(meth)acrylate, polystyrenes, polyesters, polyamides, polyimides, and polyureas.

### 3. Various additives

Note that the structured photocatalyst composition may further contain various additives such as solvents as necessary.

The solvents that can be used may be a publicly known material, and examples of the solvent include aqueous solvents such as water, organic solvents such as alcohols, and mixed solvents of these. The structured photocatalyst composition may be adjusted to a viscosity suitable for use by adding a solvent.

### Photocatalyst Coated Material

Incidentally, when a photocatalyst is employed in an actual usage, a photocatalytic layer is generally formed on a surface of a base material (e.g., a wall material, a tile, a glass (mirror), a circulation filtration apparatus, or a sanitary pottery). As the method for forming such a photocatalytic layer, for example, a method, in which a binder material is mixed with a photocatalyst to form a coating material, and the resultant coating material is applied onto the surface of the base material, is known.

Such a photocatalyst coated material including a photocatalytic layer formed on the base material in the related art has a configuration in which a photocatalytic substance is in direct contact with the base material, and the high catalytic activity of the photocatalytic substance may decompose a substance of the base material. Such a decomposition of the substance of the base material led to a deterioration of the base material, contributing to a disengagement of the photocatalytic material of the photocatalytic layer formed on the surface of the base material, from the surface of the base material.

To resolve such an issue, a method using a silane coupling agent as a binder material is proposed (WO 2016/181622 A1). Unfortunately, even such a method employs the configuration in which a photocatalytic substance comes in direct contact with the base material, thus the base material was insufficiently prevented from being deteriorated.

As a result of intensive research to achieve the object described above, the present inventors have discovered that, in a photocatalyst coated material including a base material and a photocatalytic layer formed on the base material, the photocatalytic layer includes a structured photocatalyst, the structured photocatalyst including a support of porous structure including a zeolite-type compound and at least one photocatalytic substance present in the support, the support including channels connecting with each other, and the photocatalytic substance including a metal oxide nanoparticle and being present at least at the channel of the support, thus a photocatalyst coated material, in which a direct contact between the base material and the photocatalytic substance is prevented and decomposition of a substance of the base material due to the photocatalytic substance is suppressed, can be formed, and thus favorable photocatalytic functionality can be maintained over a long period of time. Thus, the present inventors have completed the present invention based on such a discovery. The structured photocatalyst according to this embodiment will be described below in detail.

The photocatalyst coated material according to this embodiment includes a base material and a photocatalytic layer formed on the base material and containing a structured photocatalyst according to the embodiment described above.

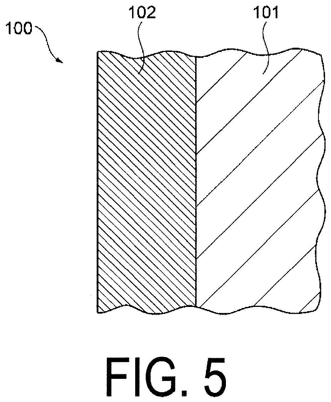

As an example of the photocatalyst coated material according to this embodiment, FIG. 5 illustrates a schematic cross-sectional view of the photocatalyst coated material according to this embodiment. As illustrated in FIG. 5, the photocatalyst coated material 100 according to this embodiment includes a base material 101 and a photocatalytic layer 102 formed on the base material 101.

Note that FIG. 5 illustrates a case where the photocatalytic layer 102 is formed directly on the surface of the base material 101, however, the photocatalytic layer 102 may be formed indirectly over the surface of the base material 101 as long as being formed over the base material 101 without being limited to the above configuration. The case where the photocatalytic layer 102 is formed indirectly over the base material 101 (not illustrated) is, for example, a case where the photocatalyst coated material includes a binding layer between the base material 101 and the photocatalytic layer 102, to fix the photocatalytic layer 102.

The photocatalytic layer contains the structured photocatalyst according to the embodiment described above, and is a coated film formed on the base material. In the photocatalyst coated material 100, a photocatalytic reaction occurs on the photocatalytic layer 102.

The photocatalytic layer 102 is required to at least include a structured photocatalyst, and may further include other components. Examples of the other components include, for example, a binder and an inorganic metal oxide excluding photocatalysts.

Such a configuration allows the photocatalytic substance to be included within the support of porous structure including a zeolite-type compound, and is formed on the base material described below as a structured photocatalyst. Therefore, on the base material, the base material is effectively prevented from coming in direct contact with the photocatalytic substance, thus enabling to prevent decomposition of a substance of the base material by the photocatalytic substance. As a result, the base material can maintain its quality without degradation over a long period of time, and superior catalytic activity can be retained without disengagement of the structured photocatalyst from a surface of the base material.

In addition, in such a structured photocatalyst, movement of the photocatalytic substance 20 within the support 10 is restricted, and thus aggregation between the photocatalytic substances 20, 20 is effectively prevented. Thus, a decrease in the effective surface area as the photocatalytic substance 20 can be effectively prevented, allowing the photocatalytic substance 20 to sustain the photocatalytic activity over a long period of time. That is, the structured photocatalyst 1 can prevent a decline in the photocatalytic activity due to aggregation of the photocatalytic substances 20, and extend the life time as the structured photocatalyst 1. In addition, the extended life time of the structured photocatalyst 1 can reduce replacement frequency of the structured photocatalyst 1, making it possible to significantly reduce an amount of waste of the structured photocatalyst 1 that has been used, and to thus achieve resource savings.

Note that, in the photocatalyst coated material, the another photocatalytic substance 30 described above illustrated in FIG. 4, which is held on the outer surface 10a of the support 10, thus comes into direct contact with the base material. This may cause the photocatalytic substance 30 to decompose the base material. Thus, a content of the at least one another photocatalytic substance 30 held on the outer surface 10a of the support 10 is preferably less than the content of the photocatalytic substance 20 present in the support 10.

### Method for Producing Photocatalyst Coated Material

The photocatalyst coated material 100 can be produced by forming the photocatalytic layer 102 on the base material 101.

The method for forming the photocatalytic layer 102 is not particularly limited, and can be performed by a publicly known method. Examples of the method include, for example, a method in which a structured photocatalyst is prepared as a coating material, and the structured photocatalyst is formed on a surface of the base material 101 by applying the coating material, by spray coating the coating material, or by immersing the base material 101 in the coating material.

### 1. Structured Photocatalyst

As a structured photocatalyst, the structured photocatalyst produced by the production method described above is used.

### 2. Base Material

Examples of the base material 101 include, but not particularly limited to, a building material, an interior material, an instrument, and a packaging material. In particular, the photocatalytic layer of the present invention is preferably formed on a building material as a base material. Here, the building material refers to a member used as an exterior member or an outdoor facility member, which are used around a building such as a residential house. Furthermore, the external member refers to a building material for finishing an outer surface facing outside of the building. Further, the outdoor facility member refers to a building material used in various outdoor facility constructions and the like externally around a building such as a residential house. In addition, examples of the outdoor facility construction include various constructions such as a fence, a gate, a fence, a handrail, a louver, a hardscape, a wall, a car shed, a garage, an earth floor, an approach, an accent wall, a pergola, a deck, a pavement material, various columns, and a barn.

In the related art, an inorganic base material was preferably used, as a base material, for photocatalyst coated materials to avoid decomposition of a substance of the base material by the photocatalytic substance. However, the photocatalyst coated material 100 according to this embodiment, in which a photocatalytic substance is included within the support as a structured photocatalyst, effectively prevents the photocatalytic substance from coming in direct contact with the base material 101, thus enabling to prevent decomposition of a substance of the base material by the photocatalytic substance. This is also effective to remove contamination on a surface of the structural body. In the photocatalyst coated material 100 according to this embodiment, an organic base material can be preferably used as well.

The base material 101 may be constituted of an organic material or an inorganic material, and examples of these materials include glass, concrete, acrylic resin, acrylic urethane resin, and tetrafluoro resin.

### 3. Other Layers

The photocatalyst coated material 100 may further include other layers (not illustrated) between the base material 101 and the photocatalytic layer 102. Examples of the other layers include a binding layer, and an ultraviolet preventing layer.

Examples of the binding layer include a layer constituted by an organic binder such as polyimide.

The photocatalyst coated material 100 according to embodiments, in which the photocatalytic substance is included within the support as a structured photocatalyst, effectively prevents the photocatalytic substance from coming in direct contact with the other layers, thus enabling to prevent decomposition of constituent substances of the other layers by the photocatalytic substance.

Descriptions will be given below by exemplifying a case where the structured photocatalyst is made into a coating material.

The coating material containing the structured photocatalyst can be produced by mixing the structured photocatalyst and the binder material, and by adding various additives as necessary.

The method of mixing is not particularly limited, and mixing can be performed by a publicly known method. For example, mixing can be performed by stirring and mixing, or by kneading using a three-roll mill or a high pressure homogenizer. Note that the structured photocatalyst includes a support including porous structure, thus, from the viewpoint of maintaining the structure in a favorable manner, the structured photocatalyst is preferably mixed by a method which does not involve a high pressure physically, such as stirring and mixing, In addition, the viscosity is preferably adjusted as appropriate, as necessary.

The resultant coating material is applied onto the base material 101, and dried and sintered as necessary, to form the photocatalytic layer 102 having a desired thickness. Examples of the drying treatment include air drying and high temperature drying.

In addition, the coating material obtained as described above may be formed into a sheet form, and the sheet may be affixed onto the base material 101 via the binding agent layer, to thus form the photocatalytic layer 102.

In the above, an example is given for a case where a structured photocatalyst is used in a coating material, and a binding agent layer may be formed on a surface of the base material 101 in advance, and a structured photocatalyst in a powdery form may be adhered onto the binding agent layer to be fixed on the base material 101, to thus form the photocatalytic layer 102.

Hereinbefore, the structured photocatalyst, the structured photocatalyst composition, a photocatalyst coated substance of embodiments according to the present invention have been described. The present invention is not limited to the embodiments described above, and various modifications and changes are possible based on the technical concept of the present invention.

For example, in the embodiments described above, the structured photocatalyst may be used as a photocatalyst dispersion liquid in which the structured photocatalyst is dispersed in a dispersion medium. As the dispersion medium, for example, a hydrophilic dispersion medium is used, such as purified water, water such as distilled water and ion exchanged water, alcohols such as methanol, ethanol, and isopropanol, or a mixture of these. Among these, water and a mixture of water and alcohol are preferred, and water is more preferred. In addition, among water, purified water, distilled water, and ion-exchanged water are preferred, and purified water is more preferred.

The method for producing the photocatalyst dispersion liquid is not particularly limited to a specific method. The photocatalyst dispersion liquid may also be prepared by adjusting the structured photocatalyst to a predetermined particle size and by dispersing the structured photocatalyst in a dispersion medium. The structured photocatalyst is adjusted to a predetermined particle size employing a bead mill provided with a hollow cylindrical container, stirring member and bead separation means arranged within the container, using spherical zirconia beads with a diameter of from 5 to 100 µm, for example. The photocatalyst dispersion liquid is produced by the following production method, for example. In a first step, the stirring member is rotated at a first rotational speed (r1) to stir with beads, the structured photocatalyst containing photocatalyst particles and a raw material dispersion liquid of pH 8 to 11 containing a dispersion medium and a basic substance, such that an average particle size (D50) of the structured photocatalyst determined by dynamic light scattering method is from 250 to 350% of the particle size of the primary particle of the structured photocatalyst. In a second step, the stirring member is rotated at a second rotational speed (r2) of from 50 to 90% of the first rotational speed (r1), such that the average particle size of the structured photocatalyst is from 150 to 250% of the particle size of the primary particle. Such a photocatalyst dispersion liquid allows a structured photocatalyst containing photocatalyst particles, to be used by applying to a target object.

The photocatalyst dispersion liquid can also be used for decomposition of aldehydes according to the embodiments described above, Examples of the aldehydes include formaldehyde, acetaldehyde, and the like.

### Examples

### Examples 1 to 96

### Synthesis of Precursor Material (A)

A silica agent (tetraethoxysilane (TEOS), available from Wako Pure Chemical Industries, Ltd.) was mixed with a surfactant serving as a molding agent to prepare a mixed aqueous solution. The pH was adjusted as appropriate, and a hydrothermal treatment was performed at from 80 to 350°C for 100 hours in a sealed container. Subsequently, the produced precipitate was filtered out, washed with water and ethanol, and was further subjected to a calcination treatment in air at 600°C for 24 hours to obtain the precursor material (A) of the types and the pore diameters listed in Tables 1 and 2. Note that the following surfactants were used depending on the types of the precursor material (A) ("the type of precursor material (A): surfactant").
- MCM-41: Hexadecyltrimethylammonium bromide (CTAB) (available from Wako Pure Chemical Industries, Ltd.)
- SBA-1: Pluronic P123 (available from BASF)

### Production of Precursor Materials (B) and (C)

Next, in accordance with a metal element (M) constituting the metal oxide nanoparticle of the type listed in Tables 1 and 2, a metal salt containing the metal element (M) was dissolved in water to prepare a metal containing aqueous solution. Note that the metal salt was used in accordance with the type of metal oxide nanoparticle ("metal oxide nanoparticle: metal salt").
- TiOₓ: Titanium tetrachloride (available from Wako Pure Chemical Industries, Ltd.)

Next, the metal containing aqueous solution was added to the precursor material (A) in a powdery form in portions in a plurality of times, and was then dried at room temperature (20°C ± 10°C) for 12 hours or longer to obtain the precursor material (B).

Note that when entry for the presence or absence of additives shown in Tables 1 and 2 is "yes", pretreatment in which an aqueous solution of polyoxyethylene (15) oleyl ether (NIKKOL BO-15 V, available from Nikko Chemicals Co., Ltd.) was added as the additive to the precursor material (A) prior to adding the metal-containing aqueous solution, and then the aqueous solution containing a metal was added as described above. Note that when the entry for the presence or absence of an additive is "no," the pretreatment with an additive as described above was not performed.

In addition, an added amount of the metal containing aqueous solution added to the precursor material (A) was adjusted to make the ratio (atomic ratio Si/M) of silicon (Si) constituting the precursor material (A) with respect to a metal element (M) contained in the metal containing aqueous solution to be the value in Tables 1 and 2.

Next, the precursor material (B) impregnated with the metal containing aqueous solution obtained as described above was subjected to a calcination treatment in air at 600°C for 24 hours to obtain the precursor material (C).

### Synthesis of Structured Photocatalyst

The precursor material (C) obtained as described above and the structure directing agent listed in Tables 1 and 2 were mixed to produce a mixed aqueous solution. Then, a hydrothermal treatment was performed, in a sealed container, under condition of from 80 to 350°C, and at pH and time listed in Tables 1 and 2. Subsequently, the produced precipitate was filtered out, washed with water, dried at 100°C for 12 hours or longer, and was further subjected to a calcination treatment in air at 600°C for 24 hours to obtain a structured photocatalyst including the support and metal oxide nanoparticle as a photocatalytic substance listed in Tables 1 and 2 (Examples 1 to 96).

### Production of Structured photocatalyst Composition

The structured photocatalyst obtained as described above in an amount of 10 parts by weight, a polyimide as a binder material (heat-resistant varnish UPIA (trade name) -AT (U-Varnish-A; trade name), available from Ube Industries, Ltd.) in an amount of 5 parts by weight, and water as a solvent in an amount of 85 parts by weight, were mixed to obtain a structured photocatalyst composition.

### Production of the photocatalyst coated material

The structured photocatalyst obtained as described above in an amount of 10 parts by weight, a polyimide as a binder material (heat-resistant varnish UPIA (trade name) -AT (U-Varnish-A; trade name), available from Ube Industries, Ltd.) in an amount of 5 parts by weight, and water as a solvent in an amount of 85 parts by weight, were mixed to obtain a structured photocatalyst composition.

Further, the resultant coating material was applied and dried on highly transmissive glass (Opti-White; registered trademark, available from Nippon Sheet Glass Co., Ltd.) as a base material to obtain a photocatalyst coated material in which a photocatalytic layer was formed on the base material.

### Comparative Example 1

In Comparative Example 1, cobalt oxide powder (II, III) having an average particle size of 50 nm or less (available from Sigma-Aldrich Japan LLC) was mixed with MFI type silicalite, to obtain a structured photocatalyst in which cobalt oxide nanoparticles as a functional substance adhered to the outer surface of the silicalite as a skeletal body.

A structured photocatalyst composition and a structured photocatalyst were also produced using the structured photocatalyst produced as above.

### Comparative Example 2

In Comparative Example 2, MFI type silicalite was synthesized in the similar manner as in Comparative Example 1 except that the step of attaching the cobalt oxide nanoparticles was omitted. A structured photocatalyst composition and a structured photocatalyst were also produced using the structured photocatalyst produced as above.

### Evaluation

Evaluations of various characteristics were conducted on the photocatalytic structural bodies of the examples described above including the support and the photocatalytic substance, and the silicalites of the comparative examples, under the conditions described below.

### [A] Cross-section observation of the structured photocatalyst

For each of the photocatalytic structural bodies of the examples described above including the support and the photocatalytic substance, and the supports themselves of the comparative examples, an observation sample was prepared by pulverization method, and a cross-section observation was conducted using transmission electron microscopy (TEM) (TITAN G2; trade name, available from FEI Company).

As a result, it was confirmed that, in the photocatalytic structural bodies of the examples described above, a photocatalytic substance was present and held within the support including silicalite or zeolite. On the other hand, in the silicalites of Comparative Example 1, the photocatalytic substance merely adhered to the outer surface of the support and was not present within the support.

Furthermore, for the photocatalytic structural bodies in which the metal oxide was titanium oxide nanoparticles (TiOx) in the examples described above, a cross-section was fabricated by FIB (focused ion beam) processing, and a section elemental analysis was conducted using SEM (SU8020, available from Hitachi High-Technologies Corporation), EDX (X-Max, available from Horiba, Ltd.). As a result, an element Ti was detected from inside the skeletal body.

It was confirmed that titanium oxide nanoparticles were present from the results of the cross-section observation using the TEM and SEM/EDX described above.

### [B] Average inner diameter of the channels of the support and average particle size of the photocatalytic substances

In the TEM image taken by the cross-section observation conducted in the evaluation [A], 500 channels in the support were randomly selected, and the respective major axis length and minor axis length were measured. The respective inner diameters were calculated from the average values (N = 500) of the measured lengths, and the average value of the inner diameters was further determined to be the average inner diameter D_{F} of the channels of the support. In addition, for the photocatalytic substance as well, 500 photocatalytic substances were randomly selected from the TEM image, and the respective particle sizes were measured (N = 500), and the average value of these was determined to be the average particle size D_{C} of the photocatalytic substances. The results are listed in Tables 1 and 2.

In addition, SAXS (small angle X-ray scattering) analysis was conducted to confirm the average particle size and dispersion of the catalytic substances. Measurements by SAXS were conducted using a Spring -8 beam line BL19B2. The resultant SAXS data were fitted with a spherical model using Guinier approximation method, and the particle size was calculated. The particle size was measured for the photocatalytic structural bodies in which the metal oxide was titanium oxide nanoparticles. Furthermore, for comparison, a commercially available titanium oxide nanoparticles (available from ISHIHARA SANGYO Co., Ltd.) was observed and measured on SEM.

As a result, in commercial products, various sizes of titanium oxide nanoparticles were randomly present in a range of particle sizes of approximately from 10 nm to 30 nm, while in the structured photocatalyst of each of the examples in which the average particle size determined from the TEM image was from 1.2 nm to 2.0 nm. Scattering peaks for particle sizes of 10 nm or less were observed in the measurement results of SAXS as well. From the measurement results of SAXS and the cross-section measurement by SEM/EDX, it was found that catalytic substances having a particle size of 10 nm or less were present within the support with a uniform particle size and in an extremely highly dispersed state.

### [C] Relationship between an added amount of the metal containing solution and an amount of metal included within the skeletal body

Photocatalytic structural bodies in which a metal oxide nanoparticle was included within the support at an added amount of atomic ratios Si/M = 50, 100, 200, 1,000 (M = Co, Ni, Fe, Cu) were produced, and then the amount of metals (mass%) included within the support of the photocatalytic structural bodies produced at the added amount described above was measured. Note that in this measurement, photocatalytic structural bodies having atomic ratios Si/M = 100, 200, and 1000 were each produced by adjusting the added amount of the metal containing solution by the same method as for the photocatalytic structural bodies having atomic ratios Si/M = 100, 200, and 1000 of Examples 1 to 384, and the structured photocatalyst having an atomic ratio Si/M = 50 was produced by the same method as for the photocatalytic structural bodies having atomic ratios of Si/M = 100, 200, and 1000, except that the added amount of the metal containing solution was varied.

The amount of metal was quantified by ICP (inductively coupled plasma) alone or in combination with ICP and XRF (fluorescence X-ray analysis). XRF (energy dispersive fluorescent x-ray analyzer "SEA1200VX," available from SSI Nanotechnology) was performed under condition of a vacuum atmosphere, an accelerating voltage 15 kV (using a Cr filter), or an accelerating voltage 50 kV (using a Pb filter).

XRF is a method for determining the amount of metal present in terms of fluorescence intensity, and XRF alone cannot determine a quantitative value (in terms of mass%). Accordingly, an amount of metal of the structured photocatalyst in which a metal had been added at Si/M = 100 was determined by ICP analysis, while an amount of metal of the structured photocatalyst in which a metal had been added at Si/M = 50 and less than 100 was calculated based on measurement results of the XRF and measurement results of the ICP.

As a result, it was confirmed that an amount of metal included within the structural body increased as the added amount of the metal containing solution was increased, at least in a range of the atomic ratio Si/M from 50 to 1000.

### [D] Performance evaluation of the structured photocatalyst

The photocatalytic activity of the photocatalytic substance was evaluated for the photocatalytic structural bodies of the examples described above and the silicalites of the comparative examples. The results are listed in Tables 1 and 2.

### (1) Photocatalytic activity

For the photocatalytic activity, an acetaldehyde decomposition test was conducted in accordance with JIS R 1701-2: 2016 to evaluate a removal performance (removal ratio) of acetaldehydes.

Specifically, 0.2 g of a structured photocatalyst was filled in an atmospheric pressure flow reactor, and an acetaldehyde containing gas serving as an odorous organic substance was supplied to the reactor while irradiating the structured photocatalyst with light. Then, the gas was brought into contact with the structured photocatalyst for three hours, and the concentration of the aldehyde after the reaction was measured. Based on the measurement results, a removal ratio of aldehyde ({(C_{A0} - C_{A})/C_{A0}} × 100) was calculated by comparing a concentration of the supplied aldehyde C_{A0} with a concentration of the aldehyde C_{A} after the reaction.

Note that a measurement of the concentration of the acetaldehyde was conducted by composition analysis of the recovered generated gas using gas chromatograph analysis (GC/MS). As the analysis device, TRACE 1310 GC (available from Thermo Fisher Scientific Inc., detector: thermal conductivity detector) were used. As the light source, a 150 W mercury lamp was used.

In this example, when the removal ratio (%) of acetaldehyde was 35% or greater, it was determined that the photocatalytic activity (resolution) was considered excellent, and rated as "A", when the removal ratio (%) was 20% or greater and less than 35%, the photocatalytic activity was considered good, and rated as "B", when the removal ratio (%) was 7% or greater and less than 20%, photocatalytic activity was considered not good, but was marginally admissible (acceptable), and rated as "C," and when the removal ratio (%) was less than 7%, the photocatalytic activity was considered poor (not acceptable), and rated as "D."

### (2) Durability (Life time)

The durability was evaluated under the following conditions:
First, the structured photocatalyst used in the evaluation (1) described above was recovered, and was then heated at 65°C for 12 hours to produce a structured photocatalyst after heating. Next, an acetaldehyde removal performance was evaluated by the same method as in the evaluation (1) described above using the resultant structured photocatalyst after heating.

Further, a degree of maintaining the removal ratio of aldehyde by the structured photocatalyst after heating was compared with the removal ratio of aldehyde by the structured photocatalyst before heating (the removal ratio obtained in the evaluation (1) described above). Specifically, a percentage (%) of the removal ratio of aldehyde by the structured photocatalyst after heating (the removal ratio obtained in this evaluation (2)) to the removal ratio of aldehyde by the structured photocatalyst before heating was calculated.

In this example, when the removal ratio of aldehyde by the structured photocatalyst after heating (the removal ratio obtained in this evaluation (2)) was maintained at 80% or greater compared to the removal ratio of aldehyde by the structured photocatalyst before heating (the removal ratio obtained by the evaluation (1) described above), the durability (heat resistance) was considered excellent, and rated as "A", when the removal ratio was maintained at 60% or greater and less than 80%, the durability (heat resistance) was considered good, and rated as "B", when the removal ratio was maintained at 40% or greater and less than 60%, the durability (heat resistance) was considered not good, but was marginally admissible (acceptable), and rated as "C," and when the removal ratio (%) was less than 40%, the photocatalytic activity was poor (not acceptable), and rated as "D."

Note that, for Comparative Example 2, the performance evaluation as in the evaluations (1) and (2) described above were also conducted. Note that Comparative Example 2 was the support itself, and did not include the photocatalytic substance. Thus, in the evaluation performance evaluation, only the support of Comparative Example 2 was filled in place of the structured photocatalyst. The results are listed in Table 2.

**[Table 1]**

| No. | Conditions of Producing Structured Photocatalyst | | | | | | | Structured Photocatalyst | | | | | Performance Evaluation | |
|---|---|---|---|---|---|---|---|---|---|---|---|---|---|---|
| | Precursor Material (A) | | Addition to Precursor Material (A) | | Hydrothermal Treatment Conditions using Precursor Material (C) | | | Support | | Photocatalytic Substance | | D_{C}/D_{F} | Photocatalytic Activity | Durability |
| | | | | | | | | Zeolite-Type Compound | | Metal Oxide Nanoparticles | | | | |
| | Type | Pore Diameter | Presence or Absence of Additives | Conversion ratio (Atomic ratio) Si/M of added amount of Metal containing Solution | Type of Structure Directing Agent | pH | Time | Structure | Average Inner Diameter of Channels D_{F} | Type | Average particle size D_{C} | | | |
| | | (nm) | | | | | (h) | | (nm) | | (nm) | | | |
| Example 1 | MCM-41 | 1.3 | Yes | 1000 | TEABr | 12 | 120 | FAU | 0.74 | TiOₓ | 0.13 | 0.2 | C | C |
| Example 2 | | | | 500 | | | | | | | 0.40 | 0.5 | C | C |
| Example 3 | | | | 200 | | | | | | | 0.66 | 0.9 | B | C |
| Example 4 | | | | 100 | | | | | | | 1.32 | 1.8 | A | B |
| Example 5 | | 2.0 | | | | | | | | | 1.98 | 2.7 | A | B |
| Example 6 | | 2.4 | | | | | | | | | 2.38 | 3.2 | A | A |
| Example 7 | | 2.6 | | | | | | | | | 2.64 | 3.6 | A | A |
| Example 8 | | 3.3 | | | | | | | | | 3.30 | 4.5 | A | A |
| Example 9 | | 6.6 | | | | | | | | | 6.61 | 8.9 | B | A |
| Example 10 | SBA-1 | 13.2 | | | | | | | | | 13.21 | 17.9 | B | A |
| Example 11 | | 19.8 | | | | | | | | | 19.82 | 26.8 | C | A |
| Example 12 | | 26.4 | | | | | | | | | 26.43 | 35.7 | C | A |
| Example 13 | MCM-41 | 1.3 | None | 1000 | | | | | | | 0.13 | 0.2 | C | C |
| Example 14 | | | | 500 | | | | | | | 0.40 | 0.5 | C | C |
| Example 15 | | | | 200 | | | | | | | 0.66 | 0.9 | B | C |
| Example 16 | | | | 100 | | | | | | | 1.32 | 1.8 | A | B |
| Example 17 | | 2.0 | | | | | | | | | 1.98 | 2.7 | A | B |
| Example 18 | | 2.4 | | | | | | | | | 2.38 | 3.2 | B | A |
| Example 19 | | 2.6 | | | | | | | | | 2.64 | 3.6 | B | A |
| Example 20 | | 3.3 | | | | | | | | | 3.30 | 4.5 | B | A |
| Example 21 | | 6.6 | | | | | | | | | 6.61 | 8.9 | C | A |
| Example 22 | SBA-1 | 13.2 | | | | | | | | | 13.21 | 17.9 | C | A |
| Example 23 | | 19.8 | | | | | | | | | 19.82 | 26.8 | C | A |
| Example 24 | | 26.4 | | | | | | | | | 26.43 | 35.7 | C | A |
| Example 25 | MCM-41 | 1.1 | Yes | 1000 | | 11 | 72 | MTW | 0.61 | | 0.11 | 0.2 | C | C |
| Example 26 | | | | 500 | | | | | | | 0.33 | 0.5 | C | C |
| Example 27 | | | | 200 | | | | | | | 0.54 | 0.9 | B | C |
| Example 28 | | | | 100 | | | | | | | 1.09 | 1.8 | A | B |
| Example 29 | | 1.6 | | | | | | | | | 1.63 | 2.7 | A | B |
| Example 30 | | 2.0 | | | | | | | | | 1.96 | 3.2 | A | B |
| Example 31 | | 2.2 | | | | | | | | | 2.18 | 3.6 | A | A |
| Example 32 | | 2.7 | | | | | | | | | 2.72 | 4.5 | A | A |
| Example 33 | | 5.4 | | | | | | | | | 5.45 | 8.9 | B | A |
| Example 34 | SBA-1 | 10.9 | | | | | | | | | 10.89 | 17.9 | B | A |
| Example 35 | | 16.3 | | | | | | | | | 16.34 | 26.8 | C | A |
| Example 36 | | 21.8 | | | | | | | | | 21.79 | 35.7 | C | A |
| Example 37 | MCM-41 | 1.1 | None | 1000 | | | | | | | 0.11 | 0.2 | C | C |
| Example 38 | | | | 500 | | | | | | | 0.33 | 0.5 | C | C |
| Example 39 | | | | 200 | | | | | | | 0.54 | 0.9 | B | C |
| Example 40 | | | | 100 | | | | | | | 1.09 | 1.8 | A | B |
| Example 41 | | 1.6 | | | | | | | | | 1.63 | 2.7 | A | B |
| Example 42 | | 2.0 | | | | | | | | | 1.96 | 3.2 | A | B |
| Example 43 | | 2.2 | | | | | | | | | 2.18 | 3.6 | B | A |
| Example 44 | | 2.7 | | | | | | | | | 2.72 | 4.5 | B | A |
| Example 45 | | 5.4 | | | | | | | | | 5.45 | 8.9 | C | A |
| Example 46 | SBA-1 | 10.9 | | | | | | | | | 10.89 | 17.9 | C | A |
| Example 47 | | 16.3 | | | | | | | | | 16.34 | 26.8 | C | A |
| Example 48 | | 21.8 | | | | | | | | | 21.79 | 35.7 | C | A |

**[Table 2]**

| No. | Conditions of Producing Structured Photocatalyst | | | | | | | Structured Photocatalyst | | | | | Performance Evaluation | |
|---|---|---|---|---|---|---|---|---|---|---|---|---|---|---|
| | Precursor Material (A) | | Addition to Precursor Material (A) | | Hydrothermal Treatment Conditions using Precursor Material (C) | | | Support | | Photocatalytic Substance | | D_{C}/D_{F} | Photocatalytic Activity | Durability |
| | | | | | | | | Zeolite-Type Compound | | Metal Oxide Nanoparticles | | | | |
| | Type | Pore Diameter | Presence or Absence of Additives | Conversion ratio (Atomic ratio) Si/M of added amount of Metal containing Solution | Type of Structure Directing Agent | pH | Time | Structure | Average Inner Diameter D_{F} of Channels | Type | Average particle size D_{C} | | | |
| | | (nm) | | | | | (h) | | (nm) | | (nm) | | | |
| Example 49 | MCM-41 | 1.0 | Yes | 1000 | TPABr | 12 | 72 | MFI | 0.56 | TiOₓ | 0.10 | 0.2 | C | C |
| Example 50 | | | | 500 | | | | | | | 0.30 | 0.5 | C | C |
| Example 51 | | | | 200 | | | | | | | 0.50 | 0.9 | B | C |
| Example 52 | | | | 100 | | | | | | | 1.00 | 1.8 | A | B |
| Example 53 | | 1.5 | | | | | | | | | 1.50 | 2.7 | A | B |
| Example 54 | | 1.8 | | | | | | | | | 1.80 | 3.2 | A | A |
| Example 55 | | 2.0 | | | | | | | | | 2.00 | 3.6 | A | A |
| Example 56 | | 2.5 | | | | | | | | | 2.50 | 4.5 | A | A |
| Example 57 | | 5.0 | | | | | | | | | 5.00 | 8.9 | B | A |
| Example 58 | SBA-1 | 10.0 | | | | | | | | | 10.00 | 17.9 | B | A |
| Example 59 | | 15.0 | | | | | | | | | 15.00 | 26.8 | C | A |
| Example 60 | | 20.0 | | | | | | | | | 20.00 | 35.7 | C | A |
| Example 61 | MCM-41 | 1.0 | None | 1000 | | | | | | | 0.10 | 0.2 | C | C |
| Example 62 | | | | 500 | | | | | | | 0.30 | 0.5 | C | C |
| Example 63 | | | | 200 | | | | | | | 0.50 | 0.9 | B | C |
| Example 64 | | | | 100 | | | | | | | 1.00 | 1.8 | A | B |
| Example 65 | | 1.5 | | | | | | | | | 1.50 | 2.7 | A | B |
| Example 66 | | 1.8 | | | | | | | | | 1.80 | 3.2 | B | A |
| Example 67 | | 2.0 | | | | | | | | | 2.00 | 3.6 | B | A |
| Example 68 | | 2.5 | | | | | | | | | 2.50 | 4.5 | B | A |
| Example 69 | | 5.0 | | | | | | | | | 5.00 | 8.9 | C | A |
| Example 70 | SBA-1 | 10.0 | | | | | | | | | 10.00 | 17.9 | C | A |
| Example 71 | | 15.0 | | | | | | | | | 15.00 | 26.8 | C | A |
| Example 72 | | 20.0 | | | | | | | | | 20.00 | 35.7 | C | A |
| Example 73 | MCM-41 | 1.0 | Yes | 1000 | TMABr | 12 | 120 | FER | 0.57 | | 0.10 | 0.2 | C | C |
| Example 74 | | | | 500 | | | | | | | 0.31 | 0.5 | C | C |
| Example 75 | | | | 200 | | | | | | | 0.51 | 0.9 | B | C |
| Example 76 | | | | 100 | | | | | | | 1.02 | 1.8 | A | B |
| Example 77 | | 1.5 | | | | | | | | | 1.53 | 2.7 | A | B |
| Example 78 | | 1.8 | | | | | | | | | 1.83 | 3.2 | A | B |
| Example 79 | | 2.0 | | | | | | | | | 2.04 | 3.6 | A | A |
| Example 80 | | 2.5 | | | | | | | | | 2.54 | 4.5 | A | A |
| Example 81 | | 5.1 | | | | | | | | | 5.09 | 8.9 | B | A |
| Example 82 | SBA-1 | 10.2 | | | | | | | | | 10.18 | 17.9 | B | A |
| Example 83 | | 15.3 | | | | | | | | | 15.27 | 26.8 | C | A |
| Example 84 | | 20.4 | | | | | | | | | 20.36 | 35.7 | C | A |
| Example 85 | MCM-41 | 1.0 | None | 1000 | | | | | | | 0.10 | 0.2 | C | C |
| Example 86 | | | | 500 | | | | | | | 0.31 | 0.5 | C | C |
| Example 87 | | | | 200 | | | | | | | 0.51 | 0.9 | B | C |
| Example 88 | | | | 100 | | | | | | | 1.02 | 1.8 | A | B |
| Example 89 | | 1.5 | | | | | | | | | 1.53 | 2.7 | A | B |
| Example 90 | | 1.8 | | | | | | | | | 1.83 | 3.2 | A | B |
| Example 91 | | 2.0 | | | | | | | | | 2.04 | 3.6 | B | A |
| Example 92 | | 2.5 | | | | | | | | | 2.54 | 4.5 | B | A |
| Example 93 | | 5.1 | | | | | | | | | 5.09 | 8.9 | C | A |
| Example 94 | SBA-1 | 10.2 | | | | | | | | | 10.18 | 17.9 | C | A |
| Example 95 | | 15.3 | | | | | | | | | 15.27 | 26.8 | C | A |
| Example 96 | | 20.4 | | | | | | | | | 20.36 | 35.7 | C | A |
| Comparative Example 1 | - | | | | | | | MFI type silicalite | 0.56 | CoOₓ | ≤ 50 | ≤ 67.6 | C | D |
| Comparative Example 2 | - | | | | | | | MFI type silicalite | 0.56 | - | - | - | D | D |

As apparent from Tables 1 and 2, the photocatalytic structural bodies (Examples 1 to 96), which were confirmed by cross-section observation to retain the photocatalytic substance within the support, were found to exhibit superior photocatalytic activity and to be also excellent in durability as a photocatalyst, compared to the photocatalytic structural bodies (Comparative Examples 1 and 2) in which the photocatalytic substance merely adheres to the outer surface of the support.

In addition, Comparative Example 2, which was the support itself and did not include any photocatalytic substance, exhibited little photocatalytic activity, and both the photocatalytic activity and the durability were inferior compared to the photocatalytic structural bodies of Examples 1 to 96.

The photocatalytic structural bodies of Comparative Examples 1 and 2, in which a photocatalytic substance adhered to only the outer surface of the support, exhibited improvement in the photocatalytic activity compared to Comparative Example 2, which was the support itself and did not have any photocatalytic substance, but were inferior in durability as a photocatalyst compared to the photocatalytic structural bodies of Examples 1 to 96.

In addition, according to the structured photocatalyst compositions of Examples 1 to 96 containing a structured photocatalyst that had such a superior photocatalytic activity, a photocatalytic layer capable of exhibiting superior anti-contamination properties can be obtained.

In addition, the structured photocatalyst compositions of Examples 1 to 96 containing the structured photocatalyst in which a photocatalytic substance was held within the support effectively prevented a direct contact between the binder material and the photocatalytic substance. Accordingly, it was found that the structured photocatalyst composition prevented a decomposition of the binder material and exhibited superior stability over a long period of time. In contrast, the structured photocatalyst compositions of Comparative Examples 1 and 2 including the structured photocatalyst in which a photocatalytic substance merely adhered to the outer surface of the support, had a configuration in which a binder material was in direct contact with the photocatalytic substance. Thus, it was confirmed that, under a circumstance where the structured photocatalyst compositions were irradiated with light, the binder material was easily decomposed due to the photocatalytic activity of the photocatalytic substance, and the stability was inferior to the structured photocatalyst compositions of Examples 1 to 96.

Furthermore, it was confirmed, by a cross-section observation, that in the structured photocatalyst contained in the photocatalytic layer constituting the photocatalyst coated materials of Examples 1 to 96, the photocatalytic substance was held within the support. Such a structured photocatalyst was found to exhibit superior photocatalytic activity and to be also excellent in durability as a photocatalyst compared to a structured photocatalyst in which a photocatalytic substance merely adhered to the outer surface of the support, contained in the photocatalytic layers constituting the photocatalyst coated materials of Comparative Examples 1 and 2.

The photocatalyst coated materials of Examples 1 to 96, which included a photocatalytic layer that included a structured photocatalyst having such a superior photocatalytic activity, exhibited superior anti-contamination properties on the surface of the photocatalytic layer.

In addition, in such a photocatalyst coated material, the photocatalytic substance was held within the support and was present above the base material, thus a direct contact between the base material and the photocatalytic substance was effectively prevented. Accordingly, it was found that the photocatalyst coated material prevented a deterioration of the base material to exhibit superior stability over a long period of time. In contrast, the photocatalyst coated materials of Comparative Examples 1 and 2 that included the structured photocatalyst in which a photocatalytic substance merely adhered to the outer surface of the support, had a configuration in which a base material was in direct contact with the photocatalytic substance, thus, it was confirmed that, under a circumstance where the photocatalyst coated materials were irradiated with light, the base material was easily deteriorated due to the photocatalytic activity of the photocatalytic substance, and the stability was inferior to the photocatalyst coated materials of Examples 1 to 96.

### Other Embodiments

A method for using a structured photocatalyst to decompose acetaldehydes, in which the structured photocatalyst includes a support of porous structure including a zeolite-type compound and a metal nanoparticle present in the support, the support including channels connecting with each other, and the metal nanoparticle being present at least at an enlarged pore portion of the channel of the support.

### Reference Signs List

1 Structured photocatalyst
10 Support
10a Outer surface
11 Channel
11a Pore
12 Enlarged pore portion
20 Photocatalytic substance
30 Photocatalytic substance
Dc Average particle size
D_{F} Average inner diameter
D_{E} Inner diameter

## Claims

1. A structured photocatalyst comprising:
a support of porous structure including a zeolite-type compound; and
at least one photocatalytic substance present in the support,
the support including channels connecting with each other, and
the photocatalytic substance being metal oxide nanoparticles and being present at least at the channels of the support.

2. The structured photocatalyst according to claim 1, wherein
the channels include an enlarged pore portion, and
the photocatalytic substance is present at least at the enlarged pore portion.

3. The structured photocatalyst according to claim 2, wherein
the enlarged pore portion causes a plurality of pores constituting any one of the one-dimensional pore, the two-dimensional pore, and the three-dimensional pore to connect with each other.

4. The structured photocatalyst according to any one of claims 1 to 3, wherein
the metal oxide nanoparticles include titanium or an alloy oxide including titanium.

5. The structured photocatalyst according to any one of claims 1 to 4, wherein
the metal oxide nanoparticles have an average particle diameter that is greater than an average inner diameter of the channels and not greater than an inner diameter of the enlarged pore portion.

6. The structured photocatalyst, for hydrodesulfurization, according to any one of claims 1 to 5, wherein
the metal oxide nanoparticles include a metal element (M) in an amount from 0.5 mass% to 2.5 mass% with respect to the structured catalyst.

7. The structured photocatalyst according to any one of claims 1 to 6, wherein
the metal oxide nanoparticles have an average particle size of from 0.1 nm to 50 nm.

8. The structured photocatalyst, for hydrodesulfurization, according to claim 7, wherein
the metal oxide nanoparticles have an average particle size ranging from 0.45 nm to 14.0 nm.

9. The structured photocatalyst according to any one of claims 1 to 8, wherein
a ratio of an average particle size of the metal oxide nanoparticles with respect to an average inner diameter of the channels is from 0.06 to 500.

10. The structured photocatalyst according to claim 9, wherein
the ratio of the average particle size of the metal oxide nanoparticles with respect to the average inner diameter of the channels is from 0.1 to 45.

11. The structured photocatalyst according to claim 10, wherein
the ratio of the average particle size of the metal oxide nanoparticles with respect to the average inner diameter of the channels is from 1.7 to 4.5.

12. The structured photocatalyst according to any one of claims 1 to 11, wherein
the channels include any one of a one-dimensional pore, a two-dimensional pore, and a three-dimensional pore defined by a framework of the zeolite-type compound, and an enlarged pore portion being different from any of the one-dimensional pore, the two-dimensional pore, and the three-dimensional pore,
the channels have an average inner diameter from 0.1 mm to 1.5 nm, and
the enlarged pore portion has an inner diameter from 0.5 mm to 50 nm.

13. The structured photocatalyst according to any one of claims 1 to 12, further comprising
at least one another photocatalytic substance held on an outer surface of the support.

14. The structured photocatalyst according to claim 13, wherein
a content of the at least one photocatalytic substance present in the support is greater than a content of the at least one another photocatalytic substance held on the outer surface of the support.

15. The structured photocatalyst according to any one of claims 1 to 14, wherein
the zeolite-type compound includes a silicate compound.

16. The structured photocatalyst according to any one of claims 1 to 15, wherein
the structured photocatalyst is dispersed in a dispersion medium.

17. A structured photocatalyst composition comprising
the structured photocatalyst described in any one of claims 1 to 16 and a binder material.

18. The structured photocatalyst composition according to claim 17, wherein
the binder material includes an organic binder.

19. A photocatalyst coated material comprising:
a base material; and
a photocatalytic layer formed on the base material, wherein
the photocatalytic layer includes the structured photocatalyst described in any one of claims 1 to 16.

20. The photocatalyst coated material according to claim 19, wherein
the base material is a building material.

21. A method for producing a structured photocatalyst, comprising:
sintering a precursor material (B), wherein a metal containing solution is impregnated into a precursor material (A) for forming a support of porous structure including a zeolite-type compound to form the precursor material (B); and
hydrothermal-treating a precursor material (C) obtained by the sintering of the precursor material (B).

22. The method for producing a structured photocatalyst according to claim 21, wherein
a non-ionic surfactant is added to the precursor material (A) by 5 to 500 mass% of the precursor material (A) prior to the sintering.

23. The method for producing a structured photocatalyst according to claims 21 or 22, wherein
the metal containing solution is added to the precursor material (A) in portions in a plurality of times prior to the sintering to impregnate the precursor material (A) with the metal containing solution.

24. The method for producing a structured photocatalyst according to any one of claims 21 to 23, wherein
when impregnating the precursor material (A) with the metal containing solution prior to the sintering,
an amount of the metal containing solution added to the precursor material (A) is adjusted to make a ratio (atomic ratio, Si/M) of silicon (Si) constituting the precursor material (A) relative to a metal element (M) included in the metal containing solution added to the precursor material (A) to be from 10 to 1000.

25. The method for producing a structured photocatalyst according to claim 21, wherein
in the hydrothermal-treating, the precursor material (C) is mixed with a structure directing agent.

26. The method for producing a structured photocatalyst according to claim 21, wherein
the hydrothermal-treating is performed under a basic condition.

27. A method for decomposing aldehydes, wherein the aldehydes are decomposed using the photocatalyst described in any one of claims 1 to 16.
